# EUROPÄISCHE PATENTSCHRIFT

(11) **EP 4 242 760 B1**
(45) Veröffentlichungstag und Bekanntmachung des Hinweises auf die Patenterteilung: **11.12.2024**
(21) Anmeldenummer: 22161598.2
(22) Anmeldetag: 11.03.2022
(51) Int. Cl.: G05B 19/418, G05B 23/02

(54) **VERFAHREN ZUR GENERIERUNG KONTEXTABHÄNGIGER INFORMATIONEN**
METHOD FOR GENERATING CONTEXT-DEPENDENT INFORMATION
PROCÉDÉ DE GÉNÉRATION D'INFORMATIONS CONTEXTUELLES

(43) Veröffentlichungstag der Anmeldung: 13.09.2023
(73) Patentinhaber: Schneider Electric Industries SAS, 92500 Rueil-Malmaison (FR)
(72) Erfinder: MENDES, Marco, 63500 Seligenstadt (DE)
(74) Vertreter: Stoffregen, Hans-Herbert

(56) Entgegenhaltungen:
- DE-A1- 102020 104 287
- US-A1- 2008 312 756
- US-A1- 2010 082 292
- US-A1- 2020 141 598

## Beschreibung

Die Erfindung bezieht sich auf ein Verfahren zur Generierung kontextabhängiger Informationen zur Information eines Benutzers über einen Zustand eines technischen Systems, wobei die kontextabhängigen Informationen des technischen Systems durch verschiedene Datenquellen bereitgestellt werden und wobei kontextabhängige Informationen eines neuen Kontextes generiert und dem Benutzer bereitgestellt werden.

Die US 2010/082292 A1 bezieht sich auf ein Verfahren zum Erzeugen eines dynamischen Kontexts für einen industriellen Prozess. Dabei werden Kontextdaten aus einem industriellen Prozess erfasst und es wird eine Anfrage empfangen, um einen berechneten Wert für die erfassten Kontextdaten durchzuführen. Es folgt ein Erkennen eines Kontextattributs durch Zugriff auf eine Prozesskategorie, die mit dem industriellen Prozess verknüpft ist und ein Erzeugen des berechneten Werts basierend auf dem erkannten Kontextattribut. Ein Prozesskontextanalysator des industriellen Prozesses bietet einem Kunden die Flexibilität, erfasste Prozessdaten anzupassen. Dadurch kann die Datenerfassung eine größere Hierarchie eines Unternehmens oder eine niedrigere Teilmenge einer Hierarchie umfassen, wobei eine entsprechende Anpassung an die Architektur erforderlich ist. Es besteht die Möglichkeit, abnormalen Betrieb oder nicht optimale Leistung zu beheben, indem erweiterte Analysen eingesetzt werden, die von der Erkennung von Attributen des flexiblen Kontexts profitieren.

Die DE 10 2020 104 287 A1 betrifft ein Verfahren und eine Vorrichtung zum Betreiben einer Produktionsanlage zur Herstellung von Werkstoffplatten, wobei die Produktionsanlage zumindest Vorrichtungen zur Beleimung des Materials, Formung eines Vlieses aus dem beleimten Material und dessen Verpressung zu Werkstoffplatten aufweist und zur Herstellung einer Werkstoffplatte Produktparameter der zu fertigenden Werkstoffplatte und Anlagenparameter der Produktionsanlage eingestellt werden, wobei die Produktionsanlage und/oder deren Vorrichtungen Sensorik zur Ermittlung von Messwerten und damit wirkverbundene Computer zur Steuerung und/oder Regelung aufweisen. Es wird vorgeschlagen, dass zumindest ein virtueller Sensor Messwerte an die Computer zur Steuerung und/oder Regelung der Produktionsanlage respektive deren Vorrichtungen übermittelt.

Die US 2020/141598 A1 betrifft ein computerimplementiertes Verfahren, umfassend: Erfassen der Umgebungsbedingungen eines Veranstaltungsortes durch ein operativ mit einem Prozessor gekoppeltes Gerät; Erstellen von Komfortprofilen für die Bewohner eines Veranstaltungsortes durch das Gerät, wobei mindestens eines der Komfortprofile eine oder mehrere Komfortpräferenzen eines Bewohners enthält, die aus dem Social-Media-Data-Mining ermittelt wurden, wobei die mehrere oder Komfortpräferenzen mindestens eine von folgenden umfassen: Temperaturpräferenz, Lautstärkepräferenz, Musikpräferenz oder Beleuchtungspräferenz; und Anpassen einer oder mehrerer Umgebungsbedingungen einer Zone des Veranstaltungsortes durch das Gerät auf Grundlage der Umgebungsbedingungen des Veranstaltungsortes und des mindestens einen der Komfortprofile.

Aus dem Stand der Technik sind des Weiteren kontextabhängige Systeme bekannt, die Informationen über ihren Kontext, d. h. ihre Umgebung, benutzen, um ihr Verhalten darauf abzustimmen. Die Basis, auf der solche Systeme arbeiten, sind Informationen, welche durch unterschiedlichste Datenquellen oder Sensoren zur Verfügung gestellt werden. Der ermittelte Kontext wird verwendet, um das Verhalten des Systems anzupassen, insbesondere das Verhalten einer Benutzerschnittstelle zur Information eines Benutzers. Dabei ist Kontext z. B. definiert als jede Art von Information, die genutzt werden kann, um eine Situation einer Entität in Interaktion mit anderen Entitäten zu charakterisieren (Anind K. Dey, u. a.: Towards a Better Understanding of Context and Context-Awareness. Graphics, Visualization and Usability Center and College of Computing, Georgia Institute of Technology, Atlanta (Georgia), 8. Juli 1999)

In Automatisierungssystemen existieren verschiedene verfügbare Informationskontexte, die jedoch nicht miteinander "verknüpft" sind. In diesem Zusammenhang sind z.B. OPC-UA "Life"-Daten eines Automatisierungsgerätes zu nennen, die möglicherweise keine direkte Beziehung zu einem Standort des Automatisierungsgerätes haben, da die verschiedenen verwendeten Informationsmodelle keinen "Kontakt" haben.

Ein weiteres Beispiel ist die Diagnose von z.B. Automatisierungsgeräten, wobei eine Vielzahl von Informationskontexten berücksichtigt werden müssen, z.B. Standort des Automatisierungsgerätes, Topologie, aktuelle Anwendung und benachbarte Automatisierungsgeräte, um sehr viel genauere Ergebnisse präsentieren zu können.

Die automatische Verarbeitung von Informationen wird zurzeit dadurch erschwert, dass Informationen getrennt sind und semantische Aspekte fehlen; denn die aus der Diagnose gewonnenen Informationen müssen von Computergeräten "verstanden" werden, d.h., die semantische Interoperabilität und die Art und Weise, wie Daten miteinander verbunden werden, muss verbessert werden.

Oft besteht auch das Problem, dass nicht alle erforderlichen Kontexte verfügbar sind. Beispiele sind Umgebungsinformationen über den Standort bzw. Raum an bzw. in dem ein Automatisierungsgerät installiert ist, wenn dieses keine Sensoren aufweist. Wenn ein Kontext nicht modelliert ist oder wenn Daten nicht verfügbar sind, wäre es vorteilhaft, wenn "geschätzte" oder "approximative" Informationen bereitgestellt werden könnten, d. h. Informationen, die ohne exaktes Messen auf Erfahrung gestützt gewonnen werden

Aus dem Stand der Technik sind zudem Verfahren bekannt, die auf der Basis von Regelmaschinen (Semantik Rule Engine SRE) mit semantischen Regeln arbeiten. Semantische Regelmaschinen ermöglichen die Implementierung dynamischer und flexibler regelbasierter Strategien und können Abfragen bearbeiten und Ergebnisse liefern, indem zusätzliches Wissen aus in Ontologien definierten Begriffen abgeleitet wird.

Semantische Regelmaschinen können zwar zur Definition von Aktionen verwendet werden, jedoch werden bisher keine Regeln für "geschätzte" Informationen generiert. Allerdings werden semantische Regelmaschinen als eine Art Datenquelle verwendet, um z.B. einen Standortkontext und davon abgeleitete Beziehungen bereitzustellen, wobei OPC-UA-Daten mit dem Standort verknüpf werden. Die Strategie für diese Zuordnung wird jedoch vorgegeben.

Es ist bisher keine Lösung bekannt, eine semantische Regelmaschine als Datenquelle für "geschätzte" bzw. "approximative" Informationen zu nutzen.

Mit OPC Unified Architecture (OPC UA) ist ein Standard für den Datenaustausch als plattformunabhängige, service-orientierte Architektur bekannt. Als neueste Generation aller Spezifikationen der Open Platform Communications (OPC) von der OPC Foundation unterscheidet sich OPC UA erheblich von ihren Vorgängerinnen insbesondere durch die Fähigkeit, Maschinendaten (Regelgrößen, Messwerte, Parameter usw.) nicht nur zu transportieren, sondern auch maschinenlesbar semantisch zu beschreiben. Folglich ist OPC-UA, oder jede andere Gerätezugriffs- und Gerätebeschreibungstechnologie, in der Lage, Geräteinformationen zu modellieren. Allerdings wird OPC-UA bisher, ähnlich wie semantische Regelmaschinen, als Informationsquelle verwendet, z.B. zur Bereitstellung eines Topologie-Kontextes und eines Diagnose-Kontextes.

Für die Diagnose eines Automatisierungsgerätes, z.B. eines Drehzahlreglers, werden aktuelle Informationen bereitgestellt, wie z.B. Fehler/Warn-Code, unterstützende Informationen/Zustände, Servicemitteilungen, Sondierungsursache (Probe cause), Abhilfe, Lösche Fehler/Warn-Code, Fehler/Wam-Historie, Testsequenzen und Warn-Gruppen. Viele der bereitgestellten Informationen sind jedoch meist gerätespezifisch. Ferner sind Informationen über Sondierungsursache (Probe cause) und Abhilfe im Geräteinformationsmodell statisch definiert. Zudem werden weitere Kontextinformationen, wie z.B. andere Geräte, Systeme und Umgebungen nicht berücksichtigt, um genauere und umfassendere Diagnoseinformationen bereitzustellen.

Davon ausgehend liegt der vorliegenden Erfindung die Aufgabe zugrunde, ein Verfahren der eingangs genannten Art derart weiterzubilden, welches die zuvor genannten Probleme des Standes der Technik behebt. Insbesondere sollen Informationen über einen neuen Kontext des technischen Systems ohne reale Datenquellen generiert werden.

Die Aufgabe wird erfindungsgemäß durch ein Verfahren mit den Merkmalen des Anspruchs 1 gelöst. Dabei werden geschätzte kontextabhängige Informationen eines neuen Kontextes, der keine reale Datenquelle darstellt, durch eine Korrelation der Datenelemente der verschiedenen Datenquellen, jedoch gestützt auf bekannte und erfahrungsbezogene Werte, generiert und dem Benutzer bereitgestellt.

Das Generieren des neuen Kontextes und zugehöriger Datenelemente umfasst die folgenden Schritte:
a) Auslösen eines Ereignisses, wie z. B. Änderung eines Diagnosewertes oder Statusabfrage des Benutzers
b) Abrufen aktueller Gerätedaten oder Lesen dieser aus der TimeSeries-Datenbank
c) ggf. Aktualisierung der Datenkorrelation
d) Triggern einer Anfrage an eine Semantic-Rule-Engine um Rückschlüsse auf bekannte Kontexte bzw. bekannte Informationen zu erhalten
e) Erstellen des neuen Kontextes geschätzter kontextbasierter Informationen basierend auf Kontext-Regeln und basierend auf dem Ergebnis der semantischen Regeln in Schritt d)
f) Zuordnung der ersten Anfrage zu den bekannten und geschätzten Informationen und Formulieren eines Ergebnisses.

Die Erfindung betrifft folglich ein Verfahren zur Ableitung neuer Kontexte geschätzter bzw. approximativer Informationen für industrielle Automatisierungsgeräte, um einen Benutzer zu unterstützen bzw. zu beraten, und zwar basierend auf Annahmen, existierenden Kontexten, deren Beziehungen und früheren Erfahrungen.

Das Verfahren basiert auf existierenden Kontexten als Datenquellen und deren Beziehung zueinander, was als Kontextualisierung von Daten bezeichnet werden kann.

Die neuen Kontexte sind nicht als direkte oder konkrete Datenquelle bzw. Informationsquelle anzusehen, sondern werden auf der Grundlage existierender Geräteinformationen und früherer Ereignisse oder Benutzererfahrungen abgeleitet.

Das erfindungsgemäße Verfahren bietet die Möglichkeit, neues Wissen aus existierendem Wissen in Form von z. B. Diagnosedaten, Topologie-Daten, Standort-Daten oder GeräteDaten abzuleiten, um einen Bediener mit verschiedenen möglichen Lösungen für ein bestimmtes Problem zu unterstützen.

Die kontextabhängigen Informationen können vorzugsweise als Informationsmodelle in Form von gerätespezifischen Daten, wie z. B. Gerätedaten, Diagnosedaten und/oder Messdaten, durch erste Datenquellen in Form von Geräten der Automatisierungstechnik, wie OPC UA-Server, bereitgestellt werden.

Ferner können ergänzend kontextbasierte Informationen, vorzugsweise als Informationsmodelle in Form von semantischen Daten, durch zweite Datenquellen in Form von Ontologien, wie Geräte-Ontologie, Standort-Ontologie, Diagnose-Ontologie, durch eine Ontologie-/Inferenzmaschine bereitgestellt werden.

Die kontextbasierten Informationen werden, vorzugsweise als Datenelemente, in den Informationsmodellen repräsentiert, wobei jedem Datenelement direkt oder indirekt eine Kontext-ID zugeordnet wird, so dass ein bestimmtes Konzept, eine bestimmte Eigenschaft oder ein bestimmtes Gerät in den verschiedenen Datenquellen tatsächlich gleich repräsentiert ist.

Die Zuordnung der Kontext-ID zu dem Datenelement erfolgt in einem OPC UA-Informationsmodell vorzugsweise durch einen Namensraum oder eine Knoten-ID, wobei eine Zuordnungstabelle verwendet wird, um den Namensraum oder die Knoten-ID in die Kontext-ID umzuwandeln.

Eine bevorzugte Verfahrensweise sieht vor, dass die Zuordnung einer Kontext-ID zu einem Datenelement in einer Ontologie für jede Instanz modelliert wird.

Als Datenquelle kann vorzugsweise eine Datenbank, insbesondere TimeSeries-Datenbank, für aktuelle und vergangenen Werte verwendet werden.

Die Korrelation der kontextbasierten Informationen der verschiedenen Datenquellen umfasst vorzugsweise folgende Schritte:
- eine Herstellung einer Verbindung zwischen einer Datenbinder-Einheit und den Datenquellen,
- eine Zuordnung zwischen kontextbasierten Informationen durch Kontextualisierung von OPC UA-Daten, indem Kontext-IDs von OPC UA-Knoten mit äquivalenten IDs in die Informationsmodelle der Ontologien aufgenommen werden,
- dynamisches Erstellen einer Datenbank basierend auf eingehenden Informationen, wobei die Kontext-ID mit Klassen der Ontologien verglichen werden und bei Übereinstimmung eine neue Beziehung in der Datenbank generiert wird.

Die Bereitstellung des neunen Kontextes geschätzter kontextabhängiger bzw. kontextbasierter Informationen umfasst vorzugsweise die Darstellung semantischer Information durch eine Benutzeroberfläche.

Vorzugsweise werden basierend auf den sicheren kontextabhängigen Informationen und den geschätzten kontextabhängigen Informationen kontextabhängige Diagnoseinformationen erzeugt.

Der Hauptanwendungsfall ist vorzugsweise die Diagnose des Funktionsstatus von Geräten, insbesondere Automatisierungsgeräten, in Verbindung mit der Generierung neuer Kontexte, die verwendet werden, um einen Benutzer über mögliche Situationen zu informieren.

Bei der Diagnose von Geräten mit Kontext werden z.B. auch mögliche Assoziationen zwischen den Geräten gemeldet. Zum Beispiel wenn einige Geräte einen schlechten Funktionszustand haben und sich im selben Raum befinden, kann der Benutzer eine Benachrichtigung über diese Geräte, einschließlich Standort erhalten, und zwar mit Vorschlägen zu möglichen Umgebungsbedingungen, was den neuen Kontext darstellt.

Wenn z.B. beide Geräte eine hohe Betriebstemperatur haben und sich im selben Raum befinden, kann eine zusätzliche Empfehlung, die auf dem oben beschriebenen Kontext basiert, darin bestehen, auch die Raumumgebung zu überprüfen, da dies zu dem beschriebenen Problem führen kann. In diesem Fall wird ein neuer Kontext "Raum mit einer variablen Temperatur" generiert, dessen Diagnose auf eine Fehlfunktion z. B. der Lüftung hinweisen kann.

Durch das erfindungsgemäße Verfahren wird die Diagnosefunktion von Geräten und Systemen verbessert werden. Die Erfindung führt nicht nur zu einer verbesserten Diagnose, sondern könnte auch auf andere Fälle angewendet werden, wie z.B. bedingte Überwachung und Vielfachkonfiguration.

Die Diagnose ist jedoch nicht der einzige Anwendungsfall für das erfindungsgemäße Verfahren.

Die Generierung kontextabhängiger Dienste kann als ein virtueller Sensor angesehen werden. Die Generierung kontextabhängiger Dienste basiert auf der Generierung eines geschätzten bzw. approximativen Kontextes, z.B. geschätzter Umgebungsinformationen, die Annahmen, verschiedene Datenquellen und Erfahrungen aus der Vergangenheit enthalten, jedoch keine realen Sensorwerte.

Die geschätzten Kontexte bzw. virtuellen Sensoren können wie folgt erstellt werden:
1. Verbindung zu Datenquellen, z. B. Kontexten, herstellen,
2. Korreliere verschiedene Kontexte miteinander, wobei eindeutige Identifikationen für dasselbe Konzept in verschiedenen Datenquellen verwendet werden.
3. Generieren neuer geschätzte Kontexte und zugehöriger Datenelemente als Antwort auf Ereignisse,
   a) Suche nach registrierten Ereignissen, die ausgelöst werden (z.B. ein schlechter Diagnosewert von einem Gerät oder eine Anfrage).
   b) Aufrufen von zugehörigem Kontext, z.B. Diagnose, von diesem Ereignis und Ausführen einer semantischen Abfrage für diesen Kontext, wobei alle Anweisungen ausgegeben werden, die diesem Kontext zugeordnet sind.
   c) Überprüfen von Anweisungen anhand von Kontextregeln, wobei dann, wenn eine Regel übereinstimmt, ein geschätzter Kontext und ein virtueller Sensorwert oder eine andere Aktion generiert wird, die dieser Regel zugeordnet ist und wobei insbesondere zu beachten ist, dass Kontextregeln auf früheren Erfahrungen und Annahmen basieren.
4. Ausgabe von Anweisungen für den Benutzer

Die Erfindung betrifft somit ein Verfahren zum Erstellen neuer Kontexte geschätzter Informationen für industrielle Automatisierungsgeräte, insbesondere wenn für diesen Kontext keine Daten bzw. Sensoren verfügbar sind. Gemäß der Erfindung ist vorgesehen, dass die neuen Kontexte aus existierenden Kontexten abgeleitet werden, z.B. aus Diagnose-Daten, Topologie-Daten, Standort-Daten der Geräte, sodann werden die unabhängigen Kontextinformationen miteinander verbunden, um einen Bediener mit verschiedenen möglichen Lösungen für ein bestimmtes Problem zu unterstützen.

Die verschiedenen Kontextinformationen und ihre Zuordnungen stellen eine neue Art von Daten bereit, d.h., die Erzeugung geschätzter Informationen auf der Grundlage von Annahmen, insbesondere beim Abfragen und Analysieren von Daten auf Schlussfolgerungen und Expertenwissen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines den Zeichnungen zu entnehmenden bevorzugten Ausführungsbeispiels.

Es zeigen:
- Fig. 1: eine schematische Darstellung eines Automatisierungssystems mit wissensbasiertem System,
- Fig. 2: eine schematische Darstellung des erfindungsgemäßen Verfahrens,
- Fig. 3: eine schematische Darstellung eines Ontologie-Schemas zur Beschreibung eines Datenelementes,
- Fig. 4: eine schematische Darstellung eines Ontologie-Schemas zur Beschreibung eines Standortes eines Gerätes,
- Fig. 5: schematische Darstellung des "Bindens" verschiedener Informationskontexte,
- Fig. 6: schematische Darstellung von Verfahrensschritten zur Generierung eines neuen Informationskontextes und
- Fig. 7: schematische Darstellung von Verfahrensschritten zur Anwendung kontextabhängiger Regeln.

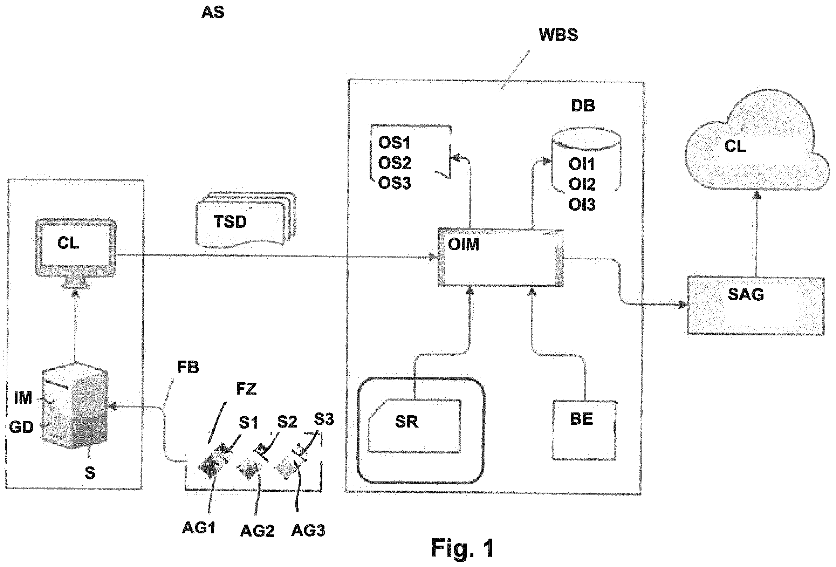

Fig. 1 zeigt schematisch ein Automatisierungssystem AS mit Geräten AG1, AG2, AG3 der Automatisierungstechnik in Form von z. B. Antrieb, Drehzahlregler, speicherprogrammierbare Steuerung, Sensor, wie Temperatursensoren, Stromsensoren, Spannungssensoren u.a., die jeweils für sich Datenquellen darstellen. Die Geräte AG1, AG2, AG3 sind z. B. gemeinsam in einer Fertigungszelle FZ angeordnet und über einen Feldbus FB, wie z. B. Modbus, mit einem Server S, vorzugsweise einem OPC-UA-Server verbunden. Der Server S ist über einen Client CL, vorzugsweise OPC-UA-Client, mit einem wissensbasierten System WBS verbunden.

Das wissensbasierte System WBS umfasst eine Ontologie-/Inferenzmaschine OIM, die Regelwissen und Faktenwissen verarbeitet. Das Regelwissen wird in Form von semantischen Regeln SR bereitgestellt. Das Faktenwissen wird durch Datenquellen in Form des Servers S, der Gerätedaten GD der Geräte über den Client CL, sowie in Form von Ontologien OS1, OS2, OS3 und zugehörige Ontologie-Instanzen OI1, OI2, OI3, die in Datenbanken DB gespeichert sind, bereitgestellt. Über eine Bedieneinheit BE kann ein Bediener Anfragen an die Ontologie-/Inferenzmaschine OIE senden und Anweisungen von dieser empfangen. Zudem kann die Ontologie-/Inferenzmaschine OIM über einen Softwareagenten SAG mit einer externen Datenquelle CL, wie z.B. einer Cloud, gekoppelt sein.

Der Server S ist eine Datenquelle, die Informationsmodelle IM der Gerätedaten GD der angeschlossenen Geräte AG1, AG2, AG3 bereitstellt. Die Informationsmodelle IM stellen Build- und Runtimedaten in Form von Parametern der Geräte und deren Beziehungen zueinander, z.B. Topologie, bereit. Über den Client CL werden aktuelle Daten der Geräte AG1, AG2, AG3 bereitgestellt.

Um einen kontinuierlichen Pool von Daten aus dem Server S zu vermeiden, können die Daten optional in einer Datenbank, z. B. einer TimeSeries-Datenbank TSD, zwischengespeichert werden, die dann als eine weitere Datenquelle für aktuelle und historische Datenwerte verwendet werden kann.

Zusätzliche Modelle können mit Hilfe der Ontologien OS1, OS2, OS3 definiert werden. Die Ontologien OS1, OS2, OS3 werden benutzt, um Datenmodelle bestimmter Informations-Domänen zu beschreiben. Eine Informations-Domäne ist beispielsweise eine Diagnose, ein Standort oder ein Gerät. Die Ontologien OS1, OS2, OS3 stellen Datenquellen dar. Beispiele für Ontologien sind je nach Anwendung eine Diagnose-Ontologie OS1, eine Standort-Ontologie OS2 sowie eine Geräte-Ontologie OS3. Durch die Ontologien OS1, OS2, OS3 werden Informationen mit semantischen Beschreibungen ergänzt, damit diese Informationen von der Ontologie-/Inferenzmaschine OIM interprätierbar sind. Die Geräte-Ontologie OS3 enthält beispielsweise bekannte Daten über Geräte, wie Gerätebeschreibungen, IP-Adressen, Kommunikationsparameter usw.

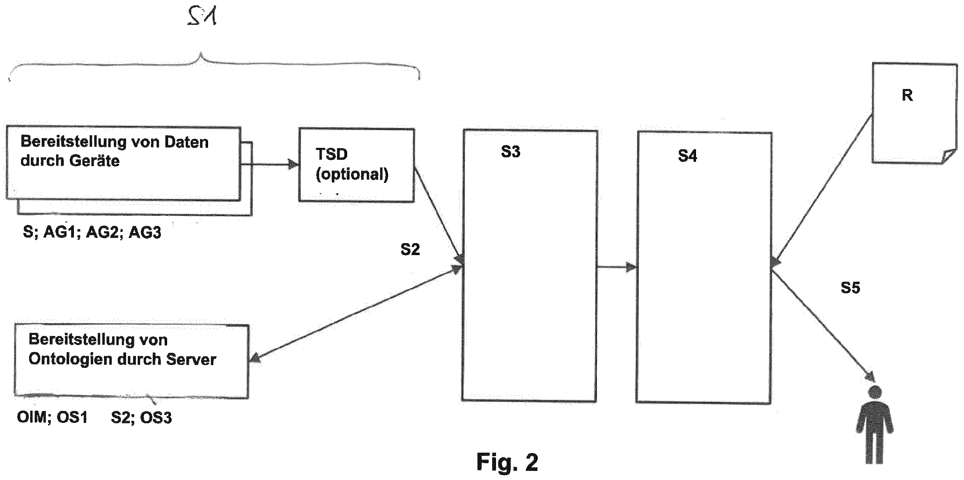

Fig. 2 zeigt rein schematisch Verfahrensschritte des erfindungsgemäßen Verfahrens.

In einem Schritt S1 werden verschiedene kontextbasierte bzw. kontextabhängige Informationen modelliert. Die Geräte AG1, AG2, AG3 stellen über den OPC-UA-Server S gerätespezifische Daten GD, wie z.B. Gerätedaten, Diagnose-Daten und/oder Messdaten bereit.

Des Weiteren werden semantische Daten der Geräte und/oder des Systems modelliert. Die semantischen Daten erweitern die Informationen über die Geräte und/oder das System. Die semantischen Daten werden über ein Informationsmodelle IM, wie OPC-UA-Topologie, oder die Ontologien OS1, OS2, OS3, wie Standort-Topologie, bereitgestellt.

Die Modellierung der verschiedenen kontextabhängigen Informationen hängt stark davon ab, welche Geräte AG1, AG2, AG3 oder Server S als Datenquelle verwendet werden und welche kontextabhängigen Informationen benötigt werden. Für OPC-UA stellt jeder Server S ein Informationsmodell IM bereit, das hauptsächlich Build- und Runtimedaten der Geräte, z. B. Parameter, sowie deren Beziehung zueinander in Form einer Topologie darstellt.

Zusätzliche Modelle werden mithilfe der Ontologien OS1, OS2, OS3 definiert und werden durch die Ontologie-/Inferenz-Maschine verfügbar gemacht. Die Ontologie-/Inferenzmaschine ist zudem eingerichtet, Anfragen durchzuführen.

In einem Schritt S2 wird eine Verbindung zu den Datenquellen in Form der Geräte AG1, AG2, AG3, dem Server S und/oder den Ontologien OS1, OS2, OS3 hergestellt.

In einem Schritt S3 werden die verschiedenen Datenquellen "verknüpft", d.h. es erfolgt eine Kontextualisierung der Daten, die weiter unten detailliert erläutert wird.

In einem Schritt S4 werden neue Kontexte generiert und zugehörige Datenelemente als Antwort auf Abfragen oder Ereignisse generiert.

In einem Schritt S5 wird ein Benutzer auf Basis der neuen Kontexte und zugehörigen Datenelemente Informiert bzw. Beraten.

Grundvoraussetzung für das erfindungsgemäße Verfahren ist, dass jedem Datenelement in den Informationsmodellen und den Ontologien eine direkte oder indirekte Kontext-ID zugeordnete wird.

In OPC-UA-Modellen kann zu diesem Zweck der Namensraum und die Knoten-ID verwendet werden, durch die ein Datenelement eindeutig identifiziert ist. Eine Zuordnungstabelle kann verwendet werden, um einen Namensraum bzw. eine Knoten-ID in eine Kontext-ID umzuwandeln.

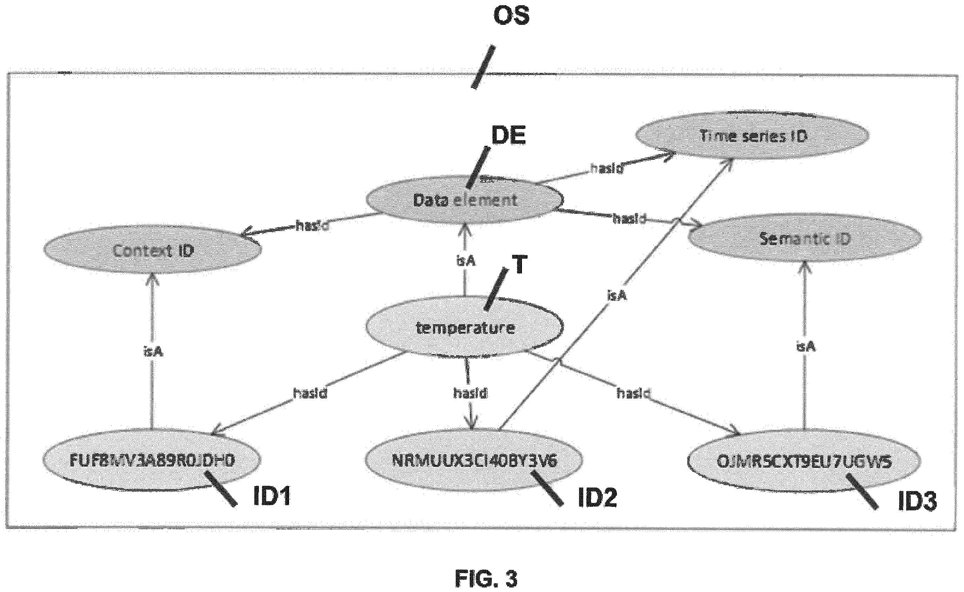

In den Ontologien OS1, OS2, OS3 muss die Zuordnung in jeder Ontologie-Instanz OI1, OI2, OI3 modelliert werden, z. B. durch Verwendung der Beziehung "hascontext-ID" bzw. "hasID", wie dies in Fig. 3 dargestellt ist.

Fig. 3 zeigt ein Ontologie-Schema OS des Begriffs Temperatur T. Die Temperatur T ist über die Relation "isA" als Datenelement DE definiert. Ferner ist dem Begriff Temperatur T über die Relationen "hasld" jeweils eine Identifizierung ID1, ID2, ID3 zugeordnet. Die Identifizierungen ID1, ID2, ID3 sind über die Relationen "isA" als Kontext-ID, Timeseries-ID sowie Semantik-ID definiert. Auch dem Datenelement DE sind über Relationen "hasId" die Kontext-ID, Timeseries-ID sowie die Semantik -ID zugeordnet.

Auf dieser Weise wird jedem Datenelement im Informationsmodell eine direkte oder indirekte Kontext-ID zugeordnet.

Sobald die Informationsmodelle und/oder Ontologien von den verschiedenen Datenquellen, wie in Schritt S1 beschrieben, bereitgestellt werden, muss eine Verbindung zu den verschiedenen Datenquellen hergestellt werden. Dies erfolgt mittels eines Daten-Binders. Gerätedaten werden z.B. direkt von dem OPC-UA-Server S oder indirekt aus der TimeSeries-Datenbank TSD abgerufen. Standortinformationen können z.B. durch Anfrage an die Ontologie-/Inferenz-Maschine erhalten werden.

Abfragen von Daten bzw. Informationen und das Zusammenführen von Daten bzw. Informationen werden nur durchgeführt, wenn Ereignisse zum Generieren oder Aktualisieren der kontextabhängigen Informationen vorhanden sind. Zum Beispiel soll die Generierung neuer Kontexte jedes Mal aktualisiert werden, wenn sich der Wert eines Funktionsstatus-Objektes ändert.

Wenn die Verbindung zu den einzelnen Datenquellen hergestellt ist, müssen die verschiedenen Datenquellen miteinander verbunden werden. Dies bedeutet, dass ein bestimmter Begriff, eine Eigenschaft oder ein Gerät in den verschiedenen Datenquellen tatsächlich gleich ist. Dies erfolgt mit Hilft der gemäß Fig. 3 den Begriffen, Eigenschaften und Geräten zugeordneten vorzugsweise identischen Kontext-IDs.

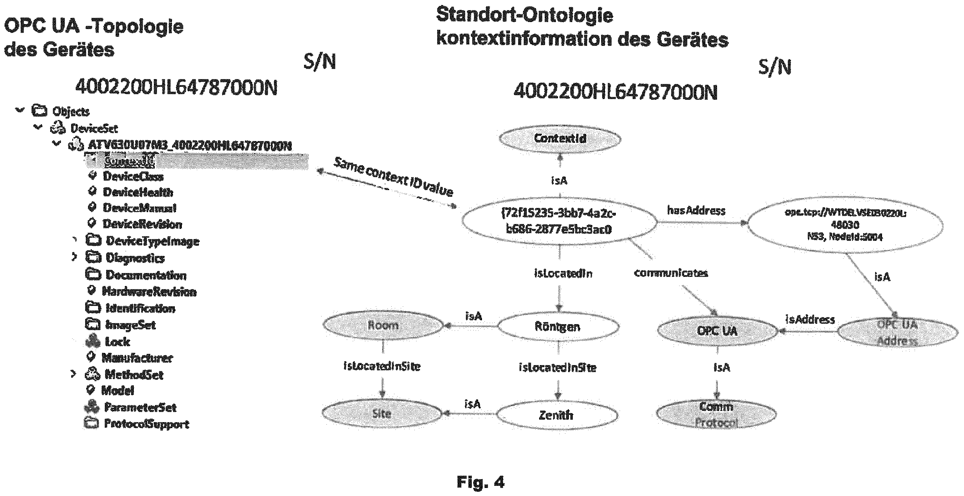

Fig. 4 zeigt eine OPC-UA-Topologie eines Gerätes mit der Seriennummer 4002200HL64787000N sowie kontextabhängige Informationen des Gerätes mit derselben Seriennummer in Form einer Standort-Ontologie. Zum Beispiel existiert in einem OPC-UA-Adressraum ein Gerät, z.B. Drehzahlregler ATV630XXX, mit einer bestimmten Kontext-ID. Dieselbe Kontext-ID wird auch in der dargestellten Standort-Ontologie verwendet. Dies bedeutet, dass beide Begriffe oder Geräte tatsächlich identisch sind, jedoch je nach Datenquelle unterschiedlich beschrieben werden: denn das OPC-UA-Informationsmodell liefert Topologie- und Diagnoseinformationen des Gerätes und die Standort-Topologie liefert Standortinformationen für dasselbe Gerät.

Fig. 4 zeigt weiter, dass in der OPC-UA-Topologie und in der Standort-Topologie derselbe Wert für die Kontext-ID verwendet wird. In der Standort-Ontologie wird dieser Wert über die Relation "isA" dem Begriff "Kontext-ID" zugeordnet. Ferner wird dem Wert über die Relation "isLocatedIn" der Begriff "Röntgen" zugeordnet. Der Begriff "Röntgen" wird über die Relation "isA" dem Begriff "Room" zugeordnet. Des Weiteren ist dem Begriff "Röntgen" über die Relation "isLocatedInSite" der Begriff "Zenith" zugeordnet. Der Begriff "Zenith" ist über die Relation "isA" dem Begriff "Site" zugeordnet.

Der Daten-Binder ist ausgebildet, die Zuordnung zwischen kontextabhängigen Informationen aus verschiedenen Datenquellen beizubehalten. Eine Kontextaktualisierung von Daten, die z.B. über OPC-UA bereitgestellt werden, kann erreicht werden, in dem Kontext-IDs in relevante OPC-UA-Knoten mit äquivalenten Identifizierungen in die Ontologie-Modelle aufgenommen werden. Dadurch wird ermöglicht, einen Topologie-Kontext, wie dieser von OPC-UA bereitgestellt wird, mit anderen durch Ontologien modulierten Kontexten zu verbinden bzw. zu verknüpfen. Dasselbe kann auch auf die Speicherung von TimeSeries-Daten angewendet werden.

Eine solche Datenbank mit kontextaktualisierten Daten wird dynamisch basierend auf den eingehenden Informationen erstellt, wobei die Kontext-IDs mit den Ontologie-Klassen verglichen werden. Bei Übereinstimmung wird eine neue Beziehung in der Datenbank generiert. Wenn bereits eine Instanz vorhanden ist, wird ein neuer Wert aktualisiert, z.B. in der TimeSeries-Datenbank. Der Abgleich eingehender Daten mit Klassen in der Ontologie kann z.B. über REGEX-Ausdrücke erfolgen.

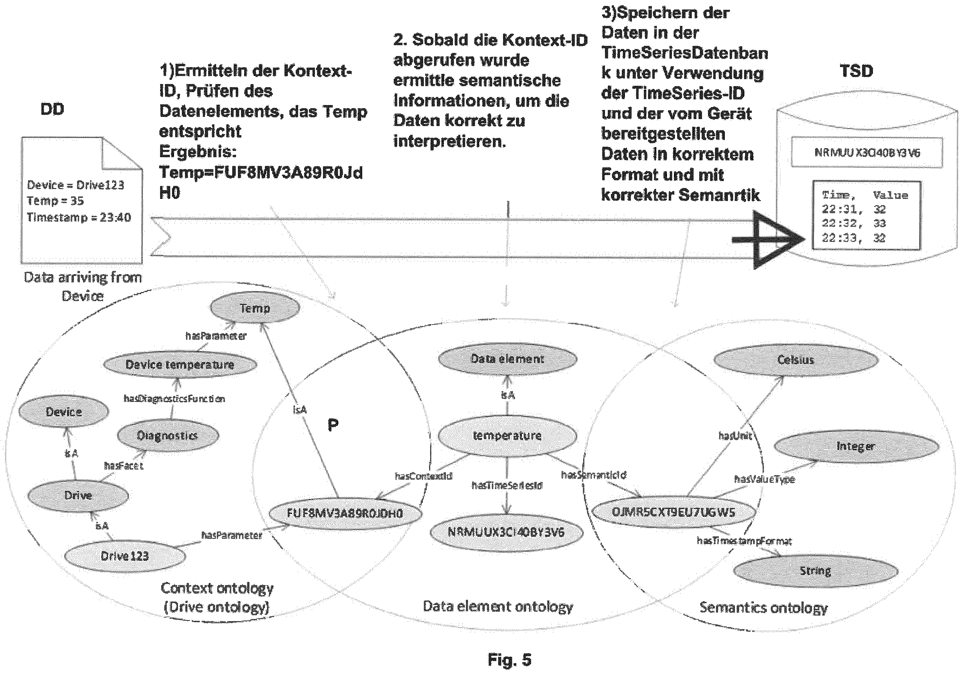

Fig. 5 zeigt beispielhaft die Erzeugung von Informationen auf der Basis von miteinander verbundenen Kontexten. Von dem Gerät AG1, z.B. einem Antrieb, werden Gerätedaten DD bereitgestellt, z.B. "Device = Drive 123", "Temp = 35" und "Timestamp = 23:40". Aus der Geräte-Ontologie ist bekannt, dass das Gerät "Drive 123" ein Antrieb ist. Der Antrieb ist ein Gerät. Ferner hat der Antrieb einen Teilaspekt "Diagnostics" mit der Funktion "Device temperature" mit dem Parameter "temp".

Das Gerät "Drive 123" hat einen Parameter "P" der eine Temperatur repräsentiert. Sodann erfolgt eine Zuordnung einer Kontext-ID. Es erfolgt eine Überprüfung mit einem Datenelement, welches mit dem Parameter "temp" korrespondiert. Dies erfolgt unter Verwendung einer Datenelement-Ontologie, wobei dem Begriff "Temperatur", der ein Datenelement ist, über die Relation "hasContextId" der Parameter "P" als Kontext-ID zugeordnet wird. Des Weiteren sind dem Begriff "Temperatur" eine Timeseries-ID und eine Semantik-ID zugeordnet.

Sobald die Kontext-ID abgerufen wurde, müssen semantische Informationen abgerufen werden, um zu wissen, wie die Daten korrekt zu interpretieren sind. Hierzu wird eine Semantik-Ontologie verwendet. Über die Semantik-ID des Begriffs "Temperatur" sind diesem Begriff eine Einheit "celsius" ein Wertetyp "integer" sowie ein Zeitstempelformat "string" zugeordnet.

Anschließend werden die Daten in der TimeSeries-Datenbank gespeichert, unter Verwendung der TimeSeries-ID und den Daten, die von dem Gerät in korrektem Format und korrekter Semantik bereitgestellt wurden.

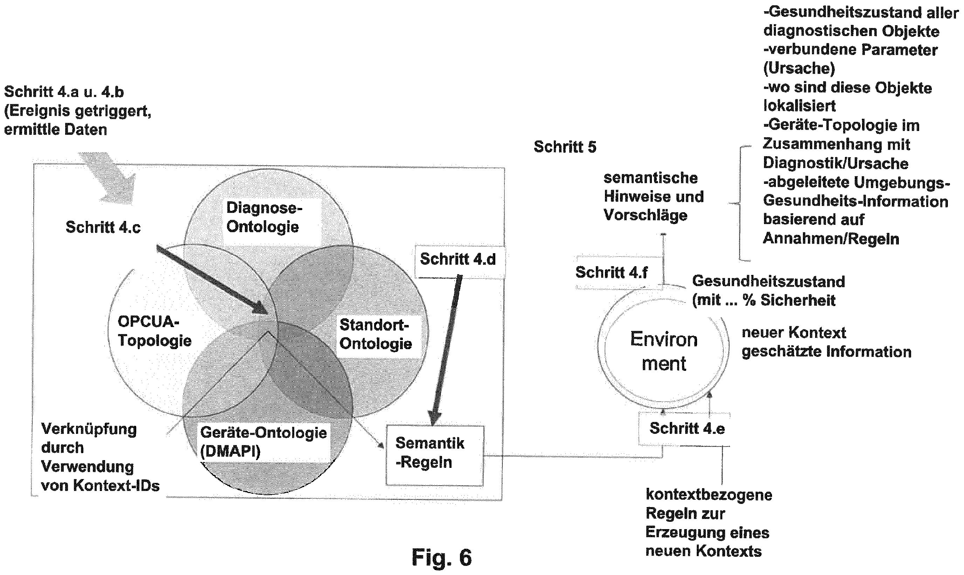

Fig. 6 zeigt im Einzelnen Verfahrensschritte der Generierung neuer Kontexte und zugehöriger Datenelemente als Reaktion auf ein Ereignis (Schritt S4). Die Generierung umfasst folgende Schritte:

| | |
|---|---|
| S4a) | Ein Ereignis wird ausgelöst. Ein Ereignis kann die Änderung eines Diagnosezustandswertes sein, der einen Sollwert überschreitet oder die Anfrage eines Benutzers, der einen Status anfordert. |
| S4b) | Aktuelle Gerätedaten werden abgerufen oder aus der Time-Series-Datenbank TSD gelesen. |
| S4c) | Gegebenenfalls Aktualisierung der Datenbindung gemäß Schritt S3. |
| S4d) | Auslösen einer Anfrage an die Ontologie-/Inferenzmaschine (Semantik-Rule-Engine), um Rückschlüsse auf bestimmte Kontexte bzw. Informationen zu erhalten. |
| | Nach diesem Schritt sind alle notwendigen Kontexte von sicheren bzw. tatsächlichen Informationen verfügbar. |
| S4e) | Generieren neuer Kontexte basierend auf kontextabhängigen Regeln und basierend auf dem Ergebnis der semantischen Regeln in Schritt 4d). |
| S4f) | Zuordnen der ursprünglichen Anfrage mit den sicheren und geschätzten Informationen und Formulierung eines Endergebnisses. |

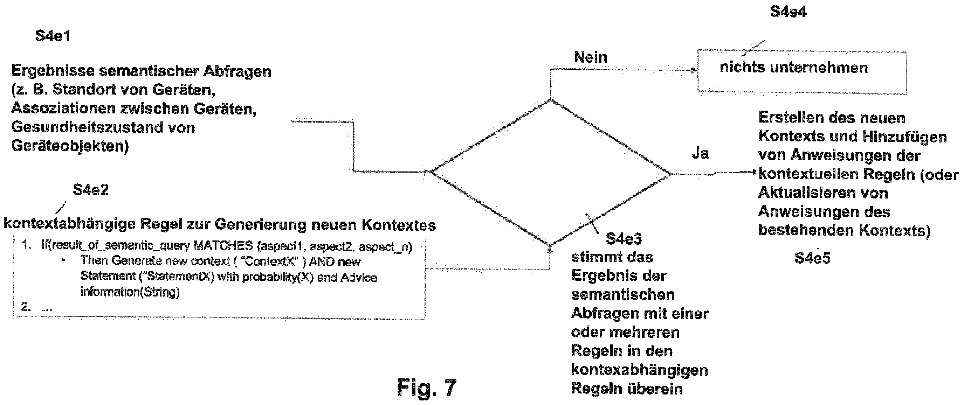

Fig. 7 zeigt rein schematisch den Verfahrensschritt S4e des Generierens neuer Kontexte basierend auf Ergebnissen einer semantischen Abfrage S4e1 und der Anwendung von Kontextregeln R gemäß Schritt S4e2.

Die semantische Abfrage gemäß Schritt S4e1 liefert beispielsweise Standort des Gerätes, Beziehungen zwischen Geräten, Funktionsstatus von Objekten des Gerätes.

In Verfahrensschritt S4e2 werden kontextbezogene Regeln R definiert, um neue Kontexte zu generieren. Eine Regel lautet z. B. "if (result semantic_query MATCHES {aspect1, aspect2, aspect_n}) Then generate new context ("ContextX") AND new Statement ("StatementX") with probalitity(X) and Advice information ("string")".

In dem Schritt S4e3 wird überprüft, ob das Ergebnis der semantischen Abfragen mit einer oder mehreren Regeln R der kontextbezogenen Regeln R übereinstimmt. Falls "Nein", wird im Schritt S4e4 nichts unternommen. Falls "Ja", wird in Schritt S4e5 ein neuer Kontext mit Datenelement aus den kontextbezogenen Regeln generiert oder Anweisung bereits existierender Kontexte werden aktualisiert.

Ein mögliches Szenario ist z. B., wenn zwei oder mehr Geräte sich in dem selben Raum (aspect) befinden und Geräte ein thermisches Diagnoseobjekt mit Zustand > 0 (aspect) aufweisen; dann erzeuge neuen Kontext ("environment") und Anweisung "health" = 1 mit Wahrscheinlichkeit von 50 %. Hinweisinformation = "Bitte überprüfe zuerst Raumumgebung".

Der Verfahrensschritt S4 soll nachfolgend in einem Beispiel näher erläutert werden:
1. Definiere ein Set von Ereignissen: E = {E₁, E₂...Eₙ}.
   Ein Ereignis kann eine Änderung eines Diagnosewertes von 0 (in Ordnung) auf 4 (außerhalb Spezifikation) oder eine Abfrage durch einen Benutzer usw. sein.
   Jedes Ereignis e ∈ E hat einen oder mehrere zugeordnete Kontexte Cₑ={C₁, C₂...Cn}. Beispielsweise steht die thermische Diagnose eines Gerätes im Kontext "Diagnose".
   Jedes Ereignis e 6 E hat ein oder mehrere zugeordnete Wert Aₑ = {a₁, a₂...a_{N}}. Der Einfachheit halber sind die einem Ereignis zugeordneten Werte alle anderen Werte im selben Kontext wie e. Wenn der Kontext von e beispielsweise "Diagnose" ist, werden alle anderen Diagnosewerte als zugeordnete Werte betrachtet, sowie ihre Beziehung erster Ordnung, die der Ursachenwert ist, der mit jedem Diagnoseobjekt verknüpft ist. Z. B. gibt es für ein thermisches Diagnoseobjekt eine Ursache, die die Betriebstemperatur ist.
2. Wenn sich ein oder mehrere Ereignisse e ∈ E ändern, erfasse deren aktuellen Wert und den aktuellen Wert aller zugeordneten Werte A aller e ∈ E die sich geändert haben. Falls notwendig, müssen diese Werte in der TimeSeries-Datenbank TSD aktualisiert werden.
   Wenn sich der Wert für die thermische Diagnose z. B. von 0 auf 4 geändert hat, muss der neue Wert (4) abgerufen werden und der Wert aller anderen Diagnoseobjekte in allen Geräten in die TimeSeries-Datenbank aktualisiert werden.
3. Erstellen einer semantischen Abfrage für alle Kontexte Cₑ die e zugeordnete sind, wobei e das Ereignis ist, das sich geändert hat. Eine mögliche Abfrage lautet: "search C".
   Im Beispiel mit der Diagnose wird ein kombinierter Kontext "Diagnose" verwendet, der die Standort-Ontologie und die OPC-UA-Topologie in einem einzigen Kontext "verbindet".
   Die Anfrage "search C" wird eine Reihe von Anweisungen S = {S₁, S₂...S_{N}} für diesen Kontext C zurückmelden. Einer Anweisung ist eine direkte oder indirekte (abgeleitete) Beziehung vom Typ "Diagnose". Beziehungen erster Ordnungen geben alle Objekte zurück, die vom Typ "Diagnose" sind wie beispielsweise "Thermal" "isA" "diagnostics", "ATV360xxx" "contains" "Thermal", "Thermal" "contains" "HealthStatus", "HealthStatus" "hasA" "Value", "Value" "is" "4", "ATV630xxx" "isLocated" "FactoryCell2", "ATV 630xxx" "isA" "Device".
4. Überprüfe, ob die vorherigen Anweisungen s ∈ S der Anfrage mit einer oder mehreren kontextbasierten Regeln R = {R₁, R₂...R_{N}} übereinstimmen. Eine Regel ist ein Algorithmus der den Satz von Anweisungen aus dem Abfrageergebnis S als Eingabe hat und war oder falsch zurückgibt, wenn S mit dem vom Algorithmus ausgedrückten Regeln übereinstimmt.
   Beispiel:
   Regel r (S) => "WENN sich zwei oder mehr Geräte in dem selben Raum befinden (dasselbe X in "islocated" X) UND Geräte eine thermisches Diagnoseobjekt haben ("Thermal" "isA" "Diagnostics", X "contains" "Thermal") mit HealthStatus-Wert > 0; DANN GEBE WAHR ZURÜCK".
5. Wenn eine oder mehrere Regeln r in R WAHR sind, dann führe die zugehörigen Aktionen mit den übereinstimmenden Regeln aus. Eine typische Aktion wäre die Generierung eines neuen Kontextes mit einer oder mehreren Eigenschaften und einer damit verbundenen Wahrscheinlichkeit.
   Beispiel:
   Generiere neuen Kontext ("environment") UND Anweisung "health" = 1 mit der Wahrscheinlichkeit von 50 %. Hinweisinformationen = "Bitte überprüfen Sie zuerst die Raumumgebung".

Schritt S5 des Verfahrens betrifft die Benutzerberatung.

Die Beratung des Benutzers hängt stark von der Benutzeroberfläche ab. Grundsätzlich werden die Informationen der vorhandenen und neuen Kontexte so formatiert, dass der Benutzer sie verstehen kann. Eine Berater-Informations-Zeichenfolge kann hier als eine Information für den Benutzer verwendet werden. Ein "Benutzer" kann auch ein zusätzliches Rechensystem sein, welches das Ergebnis der Abfrage semantisch verstehen kann.

Durch das erfindungsgemäße Verfahren können neue Kontexte geschätzter Informationen für industrielle Automatisierungsgeräte erzeugt werden, auch dann, wenn für diese Kontexte keine Daten bzw. Sensoren verfügbar sind. Die neuen Kontexte werden aus existierenden Kontexten, wie Diagnose-Daten, Topologie-Daten oder Standort-Daten der Geräte abgeleitet. Sodann werden die unabhängigen kontextabhängigen Informationen miteinander verbunden. Anschließend wird ein Bediener mit verschiedenen möglichen Lösungen für ein bestimmtes Problem durch Anweisungen unterstützt.

Die verschiedenen kontextabhängigen Informationen und deren Zuordnung stellen eine andere Wertstufe für Daten bereit, d.h., die Erzeugung geschätzter Informationen auf der Grundlage von Annahmen von insbesondere beim Abfragen und Analysieren von Daten auf Schlussfolgerungen und Expertenwissen.

In diesem Zusammenhang ist auch der Funktionsstatus (health status) eines thermischen Objekts eines Automatisierungsgerätes, wie beispielsweise eines Drehzahlreglers, zu betrachten, der in einer Fertigungszelle angeordnet ist, in der auch ein anderes Automatisierungsgerät angeordnet ist, das denselben Zustand aufweist. Beide Automatisierungsgeräte sind über OPC-UA mit einem SCADA (Supervisory Control and Data Acquisition) - System verbunden und werden in einer Prozesssteuerung zur Steuerung einiger Motoren verwendet. Für einen Benutzer wäre es sehr hilfreich, wenn dieser die Informationen über den Status der thermischen Objekte der Automatisierungsgeräte erhalten würde, sobald ein Funktionsproblem vorliegt.

Die Anwendung des erfindungsgemäßen Verfahrens in Bezug auf einen Umgebungs-Kontext soll anhand der Fig. 1 an einem Beispiel beschrieben werden. Die Automatisierungsgeräte AG1, AG2, AG3, befinden sich gemeinsam in der Fertigungszelle FZ. Ein Umgebungs-Kontext existiert nicht, da kein realer Sensor oder ein anderer realer Wert verfügbar ist, der über den Zustand der Fertigungszelle FZ informiert. Es besteht keine Möglichkeit den Zustand, z.B. die Temperatur, in der Fertigungszelle FZ zu erfassen.

Allerdings ist der Standort der Geräte AG1, AG2, AG3 bekannt und es besteht die Möglichkeit, die Betriebstemperatur der Geräte AG1, AG2, AG3 über Gerätesensoren S1, S2, S3 zu erfassen.

Hier stellt sich die Frage, ob auf der Grundlage der gemessenen Temperaturen Annahmen über den Zustand bzw. die Umgebung der Fertigungszelle FZ basierend auf den existierenden Werten, hier Temperaturwerte der Geräte, angenommen werden können.

Hier setzt die Erfindung an.
1. In einem ersten Schritt wird eine Verbindung zu den Datenquellen (Kontexten) OPC-UA-Server S und der Standort-Ontologie hergestellt. Der OPC-UA-Server stellt OPC-UA-Informationen bereit, die Temperaturwerte des Gerätes liefern. Die Standort-Ontologien stellt Standortinformationen der Geräte AG1, AG2, AG3 bereit.
2. Die verschiedenen Kontexte werden miteinander korreliert bzw. gebunden, wobei eindeutige Kontext-IDs für dieselben Begriffe in verschiedenen Datenquellen verwendet werden.
   Anschließend werden neue geschätzte Kontexte und zugehörige Datenelemente als Antwort auf Abfragen oder Ereignisse generiert.
3.1 Suche nach registrierten Ereignissen die ausgelöst werden, z.B. zumindest ein erhöhter Temperaturwert.
3.2 Abfrage des zugeordneten Kontextes, z.B. Diagnose, des Ereignisses und Durchführen einer semantischen Abfrage für diesen Kontext. Dabei werden alle Anweisungen zurückgegeben, die diesen Kontext zugeordnet sind. Z.B: "Thermal" "isA" "diagnostics", "ATV360xxx" "contains" "Thermal", "Thermal" "contains" "HealthStatus", "HealthStatus" "hasA" "Value", "Value" "is" "4", "ATV630xxx" "isLocated" "FactoryCell2", "ATV 630xxx" "isA" "Device".
3.3 Überprüfe Anweisungen anhand von Kontextregeln. Wenn eine Regel übereinstimmt, generiere geschätzten Kontext und einen virtuellen Sensorwert oder eine andere Aktion, die dieser Regel zugeordnet ist. Die Kontextregeln basieren auf früheren Erfahrungen und Annahmen. Eine Regel lautet z.B.:
   WENN sich zwei oder mehr Geräte im selben Raum befinden (dasselbe X in "isLocated" X) UND die Geräte ein Diagnoseobjekt Temperatur aufweisen ("Temperatur" "isA" "diagnostics", X "contains" "Temperature") mit Wert ! = OK ; DANN generiere neuen Kontext ("Environment") UND Anweisung "Temp" = NOK mit Sicherheit von 50 %. Hinweisinformationen = "Bitte überprüfen Sie zuerst die Raumumgebung".

## Patentansprüche

1. Verfahren zur Generierung kontextabhängiger Informationen zur Information eines Benutzers über einen Zustand eines technischen Systems, wobei die kontextabhängigen Informationen des technischen Systems durch verschiedene Datenquellen bereitgestellt werden und wobei kontextabhängige Informationen eines neuen Kontextes generiert und dem Benutzer bereitgestellt werden,
**dadurch gekennzeichnet,**
**dass** geschätzte kontextabhängige Informationen eines neuen Kontextes, der keine reale Datenquelle darstellt, durch eine Korrelation von Datenelementen der verschiedenen Datenquellen, jedoch gestützt auf bekannte und erfahrungsbezogene Werte generiert werden,
wobei das Generieren der geschätzten kontextabhängigen Informationen des neuen Kontextes und zugehöriger Datenelemente die folgenden Schritte umfasst:
a) Auslösen eines Ereignisses (Schritt S4a)
b) Abrufen aktueller Gerätedaten (Schritt S4b)
d) Triggern einer Anfrage an eine Semantic-Rule-Engine um Rückschlüsse auf bekannte Kontexte bzw. bekannte Informationen zu erhalten (Schritt S4d)
e) Erstellen des neuen Kontextes geschätzter kontextabhängiger Informationen basierend auf Kontext-Regeln und basierend auf dem Ergebnis der semantischen Regeln in Schritt d) (Schritt S4e)
f) Zuordnung der Anfrage zu den bekannten und geschätzten Informationen und Formulieren eines Ergebnisses (Schritt S4f).

2. Verfahren nach Anspruch 1,
**dadurch gekennzeichnet,**
**dass** die kontextabhängigen Informationen als Informationsmodelle in Form von gerätespezifischen Daten, wie z. B. Gerätedaten, Diagnose-Daten und/oder Messdaten, durch erste Datenquellen in Form von Geräten der Automatisierungstechnik, wie Open Platform Communications Unified Architecture - Server, OPC UA-Server, bereitgestellt werden.

3. Verfahren nach Anspruch 1 oder 2,
**dadurch gekennzeichnet,**
**dass** die kontextabhängigen Informationen als Informationsmodelle in Form von semantischen Daten durch zweite Datenquellen in Form von Ontologien, wie Geräte-Ontologie, Standort-Ontologie, Diagnose-Ontologie, durch eine Ontologie-/Inferenzmaschine bereitgestellt werden.

4. Verfahren nach Anspruch 2 oder 3,
**dadurch gekennzeichnet,**
**dass** die kontextabhängigen Informationen als Datenelemente in den Informationsmodellen repräsentiert werden, wobei jedem Datenelement direkt oder indirekt eine eindeutige Kontext-ID zugeordnet wird, so dass ein bestimmtes Konzept, eine bestimmte Eigenschaft oder ein bestimmtes Gerät in den verschiedenen Datenquellen tatsächlich gleich repräsentiert ist.

5. Verfahren nach Anspruch 4,
**dadurch gekennzeichnet,**
**dass** die Zuordnung der Kontext-ID zu dem Datenelement in einem OPC UA-Informationsmodell durch einen Namensraum oder eine Knoten-ID erfolgt, wobei eine Zuordnungstabelle verwendet wird, um den Namensraum oder die Knoten-ID in die Kontext-ID umzuwandeln.

6. Verfahren nach Anspruch 4,
**dadurch gekennzeichnet,**
**dass** die Zuordnung der Kontext-ID zu einem der Datenelemente in einer der Ontologien für jede Instanz modelliert wird.

7. Verfahren nach zumindest einem der vorhergehenden Ansprüche,
**dadurch gekennzeichnet,**
**dass** als Datenquelle eine Datenbank, insbesondere Time-Series-Datenbank, für aktuelle und vergangenen Werte verwendet wird.

8. Verfahren nach Anspruch 4,
**dadurch gekennzeichnet,**
**dass** die Korrelation der kontextabhängigen Informationen der verschiedenen Datenquellen folgende Schritte umfasst:
• eine Herstellung einer Verbindung zwischen einer Daten-Binder-Einheit und den Datenquellen,
• eine Zuordnung zwischen kontextabhängigen Informationen durch Kontextualisierung von OPC UA-Daten, indem Kontext-IDs von OPC UA-Knoten mit äquivalenten IDs in die Informationsmodelle der Ontologien aufgenommen werden,
• dynamisches Erstellen einer Datenbank basierend auf eingehenden Informationen, wobei die Kontext-ID mit Klassen der Ontologien verglichen werden und bei Übereinstimmung eine neue Beziehung in der Datenbank generiert wird.

9. Verfahren nach zumindest einem der vorhergehenden Ansprüche,
**dadurch gekennzeichnet,**
**dass** die Bereitstellung des neunen Kontextes geschätzter kontextabhängiger Informationen die Darstellung semantischer Information durch eine Benutzeroberfläche umfasst.

10. Verfahren nach zumindest einem der vorhergehenden Ansprüche,
**dadurch gekennzeichnet,**
**dass** basierend auf den sicheren kontextabhängigen Informationen und den geschätzten kontextabhängigen Informationen Diagnoseinformationen erzeugt werden.

11. Verfahren nach zumindest einem der vorhergehenden Ansprüche, **dadurch gekennzeichnet,**
**dass** das Verfahren einen Schritt
c) Aktualisierung der Datenkorrelation (Schritt S4c)
umfasst, der nach dem Schritt b) ausgeführt wird.

## Claims

1. A method for generating items of context-dependent information for informing a user about a status of a technical system, wherein the items of context-dependent information of the technical system are provided by various data sources, wherein context-dependent information of a new context are generated and provided to the user,
**characterIzed in that**
estimated context-dependent items of information of a new context, which is not a real data source, are generated by correlating the data elements of the various data sources, but based on known and experience-related values,
generating the estimated context-dependent items of information of a new context and associated data elements comprises the following steps:
d) triggering an event (step S4a)
e) retrieving current device data (step S4b)
f) triggering a query at a semantic rule engine to obtain inferences about known contexts or known items of information (step 4Sd)
g) creating the new context of items of estimated context-dependent information based on context rules and based on the result of the semantic rules in step d) (step S4e)
h) assigning the first query to the known and estimated items of information and formulating a result (step S4f).

2. The method as claimed in claim 1,
**characterized in that**
the items of context -dependent information are provided as information models in the form of device-specific data, e.g., device data, diagnostic data, and/or measurement data, by first data sources in the form of devices of automation technology, such as Open Platform Communications Unified Architecture - Servers, OPC UA servers.

3. The method as claimed in claim 1 or 2,
**characterized in that**
the context-based items of information are provided as information models in the form of semantic data by second data sources in the form of ontologies, such as device ontology, location ontology, diagnostic ontology, by an ontology/inference machine.

4. The method as claimed in claim 2 or 3,
**characterized in that**
the items of context-based information are represented as data elements in the information models, wherein a context ID is assigned directly or indirectly to each data element so that a specific concept, a specific property, or a specific device is actually represented identically in the various data sources.

5. The method as claimed in claim 4,
**characterized in that**
the assignment of the context ID to the data element takes place in an OPC UA information model by way of a namespace or a node ID, wherein an assignment table is used to convert the namespace or the node ID into the context ID.

6. The method as claimed in claim 4,
**characterized in that**
the assignment of the context ID to the data element in an ontology is modeled for each instance.

7. The method according to at least one of the preceding claims,
**characterized in that**
a database, in particular a TimeSeries database, is used as the data source for current and past values.

8. The method as claimed in claim 4,
**characterized in that**
the correlation of the items of context-based information from the various data sources comprises the following steps:
• establishing a connection between a data binder unit and the data sources,
• assigning between items of context-dependent information by contextualizing OPC UA data, **in that** context IDs of OPC UA nodes are received with equivalent IDs in the information models of the ontologies,
• dynamically creating a database based on incoming items of information, wherein the context IDs are compared to classes of the ontologies and a new relationship is generated in the database upon correspondence.

9. The method according to at least one of the preceding claims,
**characterized in that**
the provision of the new context of estimated items of context-dependent information comprises the presentation of semantic information by a user interface.

10. The method as claimed in claim 9,
**characterized in that**
items of context-dependent diagnostic information are generated based on the items of reliable or known context-dependent information and the items of estimated context-dependent information.

11. The method as claimed in claim 9,
**characterized in that**
the method performs a step
c) Actualization of the data relation (step 4c)
after the step b).

## Revendications

1. Procédé de génération d'informations contextuelles pour informer un utilisateur d'un état d'un système technique, sachant que les informations contextuelles du système technique sont fournies par différentes sources de données et sachant que des informations contextuelles d'un nouveau contexte sont générées et fournies à l'utilisateur,
**caractérisé en ce**
**que** des informations contextuelles estimées d'un nouveau contexte, qui ne représente aucune source de données réelle, sont générées par une corrélation des éléments de données des différentes sources de données, mais en se basant sur des valeurs connues et tirées de l'expérience,
sachant que la génération des informations contextuelles estimées du nouveau contexte et des éléments de données correspondants comprend les étapes suivantes :
a) Déclenchement d'un événement (étape S4a)
b) Récupération des données actuelles des appareils (étape S4b)
d) Déclenchement d'une demande adressée à un Semantic Rule Engine afin d'obtenir des déductions sur des informations ou contextes connus (étape S4d)
e) Création du nouveau contexte des informations contextuelles estimées sur la base de règles contextuelles et sur la base du résultat des règles sémantiques dans l'étape d) (étape S4e)
f) Attribution de la demande à des informations connues et estimées et formulation d'un résultat (étape S4f).

2. Procédé selon la revendication 1,
**caractérisé en ce**
**que** les informations contextuelles sont fournies comme modèles d'information sous la forme de données spécifiques aux appareils, par ex. données d'appareils, données de diagnostic et/ou données de mesure par de premières sources de données sous la forme d'appareils appartenant aux techniques d'automatisation, tels que serveur Open Platform Communications Unified Architecture - serveur OPC UA.

3. Procédé selon la revendication 1 ou 2,
**caractérisé en ce**
**que** les informations contextuelles sont fournies comme modèles d'information sous la forme de données sémantiques par de secondes sources de données sous la forme d'ontologies, telles qu'ontologie d'appareils, ontologie de sites, ontologie de diagnostics, par un moteur d'ontologie/d'inférence.

4. Procédé selon la revendication 2 ou 3,
**caractérisé en ce**
**que** les informations contextuelles sont représentées comme éléments de données dans les modèles d'information, sachant qu'un ID contextuel unique est attribué directement ou indirectement à chaque élément de données de sorte qu'un certain concept, une certaine propriété ou un certain appareil est représenté effectivement de manière identique dans les différentes sources de données.

5. Procédé selon la revendication 4,
**caractérisé en ce**
**que** l'attribution de l'ID contextuel à l'élément de données a lieu dans un modèle d'information OPC UA par un espace nom ou un ID de noeud, sachant qu'est utilisé un tableau de correspondance pour convertir l'espace nom ou l'ID noeud en ID contextuel.

6. Procédé selon la revendication 4,
**caractérisé en ce**
**que** l'attribution de l'ID contextuel à un des éléments de données est modélisée dans une des ontologies pour chaque instance.

7. Procédé selon au moins l'une des revendications précédentes,
**caractérisé en ce**
**qu'**une base de données, notamment une base Time Séries, est utilisée comme source de données pour les valeurs actuelles et passées.

8. Procédé selon la revendication 4,
**caractérisé en ce**
**que** la corrélation des informations contextuelles des différentes sources de données comprend les étapes suivantes :
• une création d'une liaison entre l'unité d'association de données et les sources de données,
• une attribution entre les informations contextuelles par contextualisation des données OPC UA en accueillant les ID contextuels des noeuds OPC UA avec ID équivalents dans les modèles d'information des ontologies,
• création dynamique d'une base de données en se basant sur des informations entrantes, sachant que les ID contextuels sont comparés aux classes des ontologies et qu'en cas de concordance, une nouvelle relation est générée dans la base de données.

9. Procédé selon au moins l'une des revendications précédentes,
**caractérisé en ce**
**que** la fourniture du nouveau contexte des informations contextuelles estimées comprend la représentation des informations sémantiques par le biais d'une interface utilisateur.

10. Procédé selon au moins l'une des revendications précédentes,
**caractérisé en ce**
**que** des informations de diagnostic sont générées sur la base des informations contextuelles certaines et des informations contextuelles estimées.

11. Procédé selon au moins l'une des revendications précédentes,
**caractérisé en ce**
**que** le procédé comprend une étape
c) Actualisation de la corrélation des données (étape S4c) qui est exécutée après l'étape b).
